# EUROPÄISCHE PATENTSCHRIFT

(11) **EP 0 808 617 B1**
(45) Veröffentlichungstag und Bekanntmachung des Hinweises auf die Patenterteilung: **31.08.2005**
(21) Anmeldenummer: 97108111.2
(22) Anmeldetag: 20.05.1997
(51) Int. Cl.: A61F 2/32

(54) **Sattelprothese**
Saddle prosthesis
Prothèse en forme de selle

(30) Priorität: 25.05.1996 DE 19621269
(43) Veröffentlichungstag der Anmeldung: 26.11.1997
(73) Patentinhaber: WALDEMAR LINK GmbH & Co. KG, 22339 Hamburg (DE)
(72) Erfinder: Nieder, Elmar Dr., D-2155 Jork (DE); Keller, Arnold, D-23863 Kayhude (DE)
(74) Vertreter: Glawe, Delfs, Moll

(56) Entgegenhaltungen:
- EP-A- 0 228 511
- EP-A- 0 300 131
- DE-A- 3 903 438
- FR-A- 2 305 961
- FR-A- 2 357 235
- GB-A- 2 005 545
- US-A- 3 869 730
- US-A- 4 054 955

## Beschreibung

Die Erfindung betrifft eine Sattelprothese für einen Hüftgelenktotalersatz, mit einem in einen Femur einsetzbaren Schaftteil und einem mit dem Schaftteil verbundenen Sattelteil, der mindestens mit zwei Freiheitsgraden gegenüber dem Schaftteil rotationsbeweglich ist und eine Sattelmulde mit einer Stützfläche für einen Teil eines Beckenknochens umfaßt.

Aus der EP-A-0 300 131 A1 der Anmelder ist bereits eine Sattelprothese bekannt, bei welcher der Sattelteil um eine im wesentlichen in Beinlängsrichtung verlaufende, gegenüber der Längsachse des Schaftteils parallelversetzte Sattelquerachse gegenüber dem Schaftteil verdrehbar, d.h. mit einem Freiheitsgrad gegenüber dem Schaftteil rotationsbeweglich ist, um eine Außen/Innenrotation des Beins zu ermöglichen. Andere Dreh- und Schwenkbewegungen des Beins, d.h. Beugung und Streckung bzw. Abduktion und Adduktion erfolgen bei dieser Sattelprothese durch eine Bewegung zwischen dem Beckenknochen und dem Sattelteil, dessen Sattelmulde flach und offen ausgebildet ist, so daß Bewegungen sowohl in Längsrichtung derselben als auch quer dazu möglich sind. In Verbindung mit dem die Außen/Innenrotation ermöglichenden Drehgelenk innerhalb der Prothese ergibt sich so eine gute Beweglichkeit des Beins, die derjenigen eines Kugelgelenks nahekommt und dazu geführt hat, daß Sattelprothesen dieser Art in der Praxis mit großem Erfolg eingesetzt werden. Im Verlauf der klinischen Erprobung und im Einsatz hat man allerdings auch festgestellt, daß der Sattelteil wegen der verhältnismäßig offenen Gestalt der Sattelmulde während der ersten Monate nach der Operation dazu neigt, aus seinem knöchernen Lager auszuwandern oder herauszuspringen. Dieses Phänomen tritt später nicht mehr auf, da sich dann in der Regel eine dichte Gewebekapsel um den metallischen Sattelteil herum ausbildet, die zudem in vielen Fällen verknöchert. Infolge der verhältnismäßig kleinen Stütz-oder Auflagefläche des Beckenknochens auf dem Sattelteil kommt es in geringem Umfang auch zu aufwärtsgerichteten Migrationsbewegungen des Sattelteils im Beckenknochen oder zu einem Bruch des Beckenknochen oberhalb des Sattels aufgrund einer Überlastung des Knochens.

Weiter wurde in der genannten Druckschrift auch eine Sattelprothese der eingangs genannten Art vorgeschlagen, welche zusätzlich ein unterhalb des Sattelteils zwischen diesem und dem Schaftteil angeordnetes Kugelgelenk aufweist (Fig. 17), das in begrenztem Maß Rotationsbewegungen um weitere Achsen zuläßt, die gegenüber der Sattelquerachse geneigt sind, so daß der Sattelteil mit drei Freiheitsgraden gegenüber dem Schaftteil rotationsbeweglich ist. Die übereinanderliegende Anordnung zweier Gelenke, d.h. zum einen des Kugelgelenks und zum anderen des Gelenks zwischen dem offen gestalteten Sattelteil einerseits und dem Beckenknochen andererseits hat jedoch eine Instabilität des Sattelteils zur Folge, die zu seinem Verkippen führen kann und deshalb einen praktischen Einsatz einer derartigen Sattelprothese verhindert hat.

Ausgehend hiervon liegt der Erfindung die Aufgabe zugrunde, eine Sattelprothese der eingangs genannten Art zu entwickeln, die eine Instabilität oder ein Verkippen des Sattelteils verhindert und es gleichzeitig ermöglicht, einem Auswandern oder Herausspringen des Beckenknochens aus dem Sattelteil und/oder einer Aufwärtsmigration des Sattelteils im Beckenknochen und/oder einem Bruch desselben oberhalb des Sattelteils entgegenzuwirken.

Diese Aufgabe wird erfindungsgemäß dadurch gelöst, daß mindestens eine der Rotationsbewegungen des Sattelteils gegenüber dem Schaftteil beim Beugen/Strecken und/oder bei der Abduktion/Adduktion des Oberschenkels um eine oberhalb der Stützfläche des Sattels liegende Schwenk- oder Drehachse erfolgt. Der Erfindung liegt der Gedanke zugrunde, den Sattelteil nicht wie beim Stand der Technik unterhalb des Sattelteils drehbar abzustützen, sondern ihn oberhalb von dessen Stützfläche, d.h. der Gewichtskräfte aufnehmenden Auflagefläche des Beckenknochens im Sattelteil schwenkbar aufzuhängen, so daß die Lastübertragung der Gewichtskräfte vom Beckenknochen auf den Sattelteil unterhalb der Aufhängung erfolgt, wodurch beim Abstützen eines Teils des Körpergewichts über die Prothese der Sattelteil entsprechend der Richtung der einwirkenden Gewichtskräfte stets die stabilste Lage einnimmt, so daß ein Verkippen unmöglich ist, ohne daß Dreh- oder Schwenkbewegungen um die besagte Dreh- oder Schwenkachse beeinträchtigt werden. Durch die im Sattelteil vorgesehene Aufhängung werden die Voraussetzungen für Dreh- und Schwenkbewegungen des Oberschenkelknochens und eine sichere Kraftübertragung geschaffen. Damit der Sattelteil nicht aufwärts migriert oder ein Bruch des Bekkenknochens zustande kommt, muß der Sattel möglichst breit sein, um die Last über eine große Fläche übertragen zu können. Ferner sollten die beiden Sattelhörner nur wenig divergieren, damit der Sattel nicht aus der Mulde im Beckenknochen herauswandern oder - springen kann. Beides - eine breite Auflagefläche und wenig divergierende Sattelhörner eines Sattels - würden bei der aus dem Stand der Technik bekannt gewordenen Sattelprothese zu einer Einschränkung der Beweglichkeit zwischen Knochen und Sattelteil führen. Um diese Einschränkung zu kompensieren, ohne auf eine breite Auflagefläche und wenig divergierende Sattelhörner verzichten zu müssen, müssen alle drei Freiheitsgrade der Bewegung, welche von der Sattelprothese übertragen werden müssen und eigentlich nur durch ein Kugelgelenk ermöglicht werden, aus der Artikulation zwischen Knochen und Sattelteil heraus und in die prothetische Konstruktion hinein verlagert werden, ohne daß diese dadurch instabil wird.

Prinzipiell ist es denkbar, nur eine der für die Beugung und Streckung bzw. die Abduktion und Adduktion des Oberschenkels erforderlichen Dreh- oder Schwenkachsen, vorzugsweise eine zwischen den gegenüberliegenden Sattelhörnern hindurch verlaufende Schwenkachse in die Prothese selbst zu verlegen und die andere auf der Oberfläche der Sattelmulde zu belassen, wobei sie sich vorzugsweise von einem der beiden Sattelhörner quer über die Sattelmulde zum anderen Sattelhorn erstreckt. Damit ließe sich zum einen die Stützfläche, auf der sich der Beckenknochen auf dem Sattelteil abstützt erheblich vergrößern, um dadurch die Wahrscheinlichkeit einer Aufwärtsmigration und/oder eines Bruchs des Beckenknochens oberhalb des Sattelteils zu verringern, zum anderen könnte die Divergenz der Sattelhörner verkleinert werden, um eine bessere Führung eines Randteils des Beckenknochens im Sattel zu erhalten, um dadurch die Wahrscheinlichkeit eines postoperativen Auswanderns oder Herausspringens des Beckenknochens aus der Sattelmulde zu verringern. Bei einer derartigen schwenkbaren Abstützung des Beckenknochens im Sattelteil und einer schwenkbaren Aufhängung des Sattelteils am Schaftteil wäre trotz der Einschränkung der Beweglichkeit durch den Sattel mit breiterer lastaufnehmender Fläche und weniger divergierenden Sattelhörnern im Gelenk zwischen Sattel und Knochen bei einer im wesentlichen senkrechten Ausrichtung der beiden Achsen eine gute Beinbeweglichkeit sowohl bei der Beugung/Streckung als auch bei der Abduktion/Adduktion sowie Kombinationen dieser zwei Bewegungen gegeben. Es ist jedoch auch möglich, nur diejenige Schwenkachse, die sich quer über die Sattelmulde von einem Sattelhorn zum anderen erstreckt, in die Prothese selbst zu verlegen und die zwischen den einander gegenüberliegenden Sattelhörnern hindurchverlaufende Schwenkachse auf der Oberfläche der Sattelmulde zu belassen.

Eine bevorzugte Ausgestaltung der Erfindung sieht jedoch vor, beide der für eine kombinierte Beugung/Streckung und Abduktion/Adduktion erforderlichen zwei Dreh- oder Schwenkachsen in die Prothese zu verlegen, um dadurch eine weitere Vergrößerung der lastaufnehmenden Stützfläche und/oder Verengung der zwischen den Sattelhörnern gelegenen Sattelmulde mit den bereits genannten Konsequenzen zu erreichen. Besonders bevorzugt wird eine kardanische Aufhängung des Sattelteils, bei welcher der Sattelteil vorzugsweise in einem Ringsegment verschwenkbar gelagert ist, welches seinerseits unabhängig davon in einer Gabel eines Halteteils drehbar gelagert ist. Diese kardanische Aufhängung gestattet es zum einen, den Schaftteil und damit den Oberschenkel für kombinierte Beuge/Streckbewegungen und Abduktions/Adduktionsbewegungen mit zwei Freiheitsgraden und mit verhältnismäßig großen Schwenkwinkeln rotatorisch gegenüber dem Sattelteil zu verschwenken, und ermöglicht es zum anderen, bei der Operation den Sattelteil in einer solchen Ausrichtung mit dem Beckenknochen in Eingriff zu bringen, die sich mit der Anatomie des Patienten, beispielsweise einem teilweise zerstörten Beckenknochen, am besten vereinbaren läßt.

Gemäß einer weiteren bevorzugten Ausgestaltung der Erfindung ist der Halteteil mit der Gabel zusätzlich gegenüber dem Schaftteil um eine im wesentlichen mit der Beinlängsachse zusammenfallende Rotationsachse drehbar, um auch eine Außen/Innenrotation des Oberschenkels gegenüber dem Becken zu ermöglichen und dem Patienten eine Beinbeweglichkeit zu verleihen, die in weitem Umfang derjenigen des natürlichen Hüftgelenks entspricht.

Grundsätzlich kann der Beckenknochen trotz der drei rotatorischen Freiheitsgrade innerhalb der Prothese noch begrenzt im Sattelteil verschwenkbar sein, um ggf. die durch die Konstruktion der Prothese vorgegebenen begrenzten Schwenkwinkel zu vergrößern. Die Verschwenkbarkeit des Beckenknochens im Sattelteil wird beispielsweise erreicht, indem man einen größeren Zwischenraum zwischen dem Beckenknochen und gegenüberliegenden Innenflächen der Sattelhörner vorsieht und/oder deren Oberflächen poliert, um ein Anwachsen von Knochen zu verhindern. Andererseits ist es aber nunmehr auch möglich, den Beckenknochen durch eine entsprechende Führung oder Fixation im Sattelteil weitgehend oder völlig zu immobilisieren, um ein postoperatives Auswandern oder Herausspringen des Beckenknochens aus dem Sattelteil sicher zu verhindern.

Die Führung kann zweckmäßig einander gegenüberliegende Führungsflächen umfassen, die gegen entgegengesetzte Knochenoberflächen des Beckenknochens anliegen und durch die Stützfläche verbunden sind, auf der sich der Beckenknochen im Sattelteil abstützt. Die Führung ist vorzugsweise rinnenförmig ausgebildet und weist einen im wesentlichen U-förmigen Querschnitt auf, wobei die einander zugewandten Innenseiten der Sattelhörner die Führungsflächen bilden.

Es ist auch denkbar, den Sattel anatomiegerecht, eventuell nach Anfertigung eines naturgetreuen Beckenmodells, so zu gestalten, daß die Innenflächen des Sattels überall dem Knochen anliegen und ferner den Sattel mit einer Oberfläche zu bekleiden, an der Knochen anwachsen kann. Die Fixation erfolgt im letzteren Fall bevorzugt durch Ausbildung von unebenen Knocheneinwachsoberflächen auf den gegen den Knochen anliegenden Innenseiten der Sattelhörner und/oder auf der Stützfläche, um eine dauerhafte Verbindung zwischen dem Knochen und dem Sattelteil zu schaffen. Alternativ ist es auch denkbar, den auf der Stützfläche abgestützten und in die Führung eingreifenden Randteil des Beckenknochens mittels Knochenzement oder mit Schrauben am Sattelteil zu verankern, wobei die letzteren zweckmäßig in Querbohrungen eingesetzt werden, die durch eines der Sattelhörnern hindurchführen und auf der gegenüberliegenden Seite der Sattelmulde als eine fluchtende Sacklochbohrung mit Innengewinde enden.

Um beim Zusammenwirken zwischen der Prothese und dem Beckenknochen möglichst große Schwenkwinkel zu erhalten, sieht eine vorteilhafte Ausgestaltung der Erfindung vor, daß sich eine der beiden Dreh- oder Schwenkachsen der kardanischen Aufhängung parallel zu einer Längsachse der Sattelmulde zwischen den beiden einander gegenüberliegenden Sattelhörnern des Sattelteils hindurch erstreckt, vorzugsweise in einer Symmetrieebene, die den Sattelteil in zwei Hälften mit jeweils einem Sattelhorn unterteilt, während die andere Schwenk- oder Drehachse vorzugsweise senkrecht dazu die beiden Sattelhörner quer zur Längsachse der Sattelmulde durchsetzt und zweckmäßig ebenfalls in einer Symmetrieebene des Sattelteils liegt. Die Dreh- oder Schwenkachsen verlaufen vorzugsweise durch den Schwerpunkt des Sattelteils oder kreuzen sich oberhalb desselben, um Dreh- oder Schwenkbewegungen um eine oder beide Achsen infolge des Eigengewichts des Sattelteils zu vermeiden.

Prinzipiell müssen sich die beiden Dreh- oder Schwenkachsen und die Rotationsachse untereinander nicht unbedingt in einem Punkt rechtwinklig schneiden, um die gewünschten drei Freiheitsgrade der Bewegung zu erhalten. Eine weitere vorteilhafte Ausgestaltung der Erfindung sieht jedoch eine cartesische Ausrichtung der drei Achsen vor, da in diesem Fall der Bewegungsablauf beim Verschwenken und/oder Verdrehen des Beins am ehesten dem natürlichen Bewegungsablauf im anatomischen Hüftgelenk entspricht.

Um die Baugröße der Prothese im Bereich des Sattelteils und seiner Aufhängung klein zu halten, sieht eine weitere vorteilhafte Ausgestaltung vor, die Verschwenkbarkeit des Sattelteils um eine der beiden Dreh- oder Schwenkachsen dadurch zu gewährleisten, daß ein Ringsegment in einer daran angepaßten gleichfalls ringförmigen äußeren Führungsnut auf der Unterseite des Sattelteils verschiebbar gelagert ist, während die andere der beiden Dreh- oder Schwenkachsen durch ein Drehlager festgelegt wird, in dem das Ringsegment gegenüber der Gabel des Halteteils drehbar gelagert ist. Zweckmäßig sind die beiden von der Führungsnut einerseits und dem Drehlager andererseits festgelegten Dreh- oder Schwenkachsen zueinander senkrecht. Bevorzugt ist die Führungsnut konzentrisch zu der in Längsrichtung der Sattelmulde verlaufenden Dreh- oder Schwenkachse in einer kugelig gerundeten Außenfläche des Sattelteils ausgespart, wobei sie sich zweckmäßig über einen Winkel von 260 bis 310 Grad und vorzugsweise von 270 bis 300 Grad erstreckt, während sich das Ringsegment vorzugsweise über einen Umfangswinkel von etwa 180 Grad erstreckt, so daß sich das Ringsegment aus einer Mittellage nach beiden seiten um etwa 45 bis 60 Grad gegenüber dem Sattelteil verschwenken läßt.

Vorzugsweise besteht die erfindungsgemäße Sattelprothese aus mehreren lösbar miteinander verbundenen Teilen, und zwar dem Sattelteil, dem in der Führungsnut des Sattelteils verschiebbaren Ringsegment, dem Halteteil mit der Gabel, in der das Ringsegment aufgehängt ist, einem um die Rotationsachse drehbar mit einem Schaft des Halteteils verbundenen Zwischenstück für einen Längenausgleich im Hinblick auf die unterschiedliche Anatomie von Patienten sowie die unterschiedliche Position der Anbringung des Sattelteils, sowie dem Schaftteil, auf dem das Zwischenstück vorzugsweise starr befestigt ist.

Im folgenden wird die Erfindung anhand eines in der Zeichnung dargestellten Ausführungsbeispiels näher erläutert. Es zeigen:

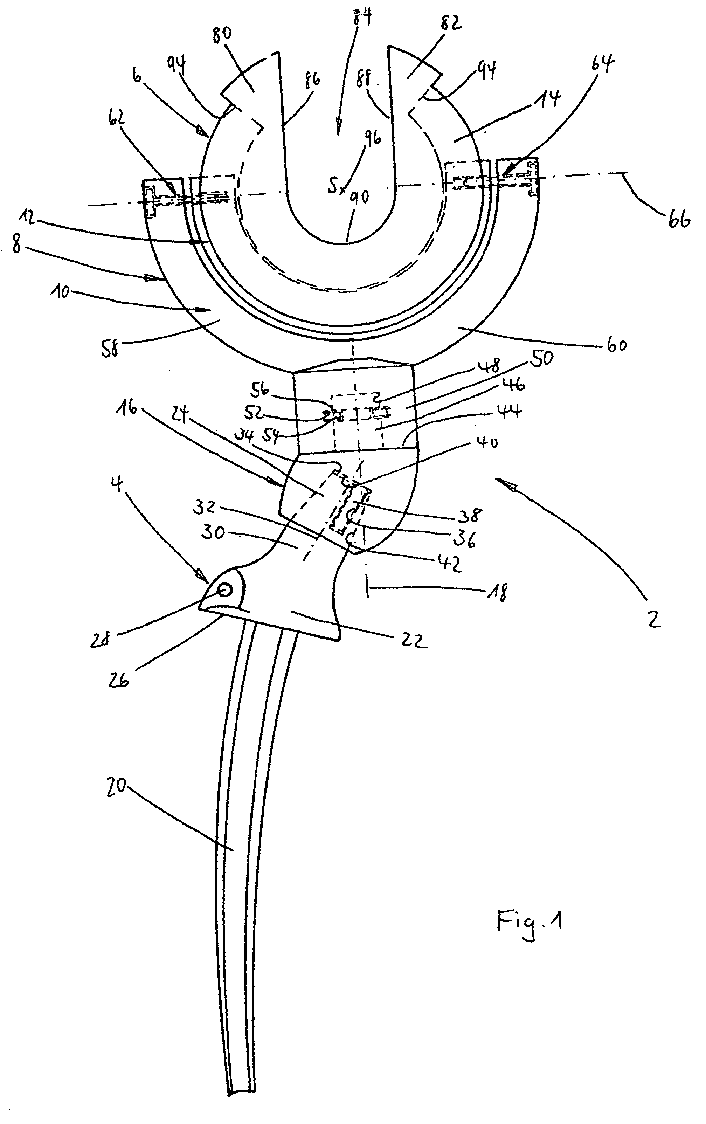

Fig. 1: eine Vorderseitenansicht einer bevorzugten Ausführungsform einer erfindungsgemäßen Sattelprothese;
Fig. 2: eine teilweise geschnittene Vorderseitenansicht des Halteteils der Sattelprothese;
Fig. 3: eine Draufsicht von oben auf den Halteteil;
Fig. 4: eine Seitenansicht des im Halteteil aufgehängten Sattelteils;
Fig. 5: eine Seitenansicht des Sattelteils;
Fig. 6: einen Schnitt durch den Sattelteil entlang der Linie 5-5 der Fig. 6.

Die in der Zeichnung dargestellte Sattelprothese 2 umfaßt im wesentlichen ein Schaftteil 4 und einen kardanisch aufgehängten Sattelteil 6, wobei die kardanische Aufhängung des Sattelteils 6 im wesentlichen aus einem gabelförmigen Halteteil 8 und einem in der Gabel 10 des Halteteils 8 drehbar gelagerten Ringsegment 12 besteht, das in einer Führungsnut 14 auf der Unterseite des Sattelteils 6 gleitend gelagert ist. Die Sattelprothese 2 umfaßt weiter ein auf den Schaftteil 4 aufgesetztes Zwischenstück 16, auf dem der Halteteil 8 um eine im wesentlichen mit einer Beinlängsachse zusammenfallende Rotationsachse 18 drehbar gelagert ist.

Der Schaftteil 4 besteht im wesentlichen aus einem langgestreckten Schaft 20, einem am oberen Ende des Schaftes 20 angeordneten Kragen 22 und einem schräg nach oben über den Kragen 22 überstehenden kegelstumpfförmigen Befestigungszapfen 24 für das Zwischenstück 16, wobei die Teile 20, 22 und 24 einstückig miteinander verbunden sind.

Der Schaft 20 ist in einen entsprechend präparierten Markhöhlenkanal eines Femur (nicht dargestellt) einführbar und mit Knochenzement oder zementlos mit einer Preßpassung und Knocheneinwachsoberflächen darin verankerbar, wobei sich der Kragen 22 mit einer dem Schaft 20 zugewandten ebenen Unterseite 26 auf dem entsprechend resezierten oberen Ende des Femur abstützt. Der Schaft 20 ist entsprechend der Krümmung des Markhöhlenkanals in lateraler Richtung etwas gekrümmt und verjüngt sich in Richtung seines freien Endes.

Der Kragen 22 weist eine seitliche Ausziehöse 28 auf, die das Herausziehen des während der Präparation des Markhöhlenkanals probeweise in diesen eingeführten Schaftes 20 erleichtert. Der Kragen 22 verjüngt sich vom Schaft 20 weg nach oben zu, wobei er in einen im wesentlichen zylindrischen Mittelteil 30 übergeht, dessen Durchmesser dem Durchmesser des unteren breiteren Endes des kegelstumpfförmigen Befestigungszapfens 24 entspricht.

Der sich vom Kragen 22 weg nach oben zu verjüngende Befestigungszapfen 24 weist eine Mittelachse 32 auf, die in einer Lateralebene unter einem Winkel von etwa 35 Grad gegenüber der Rotationsachse 18 geneigt ist. An seinem freien Ende ist der Befestigungszapfen 24 mit einer ebenen Stirnfläche 34 versehen, in die eine zur Mittelachse 32 des Befestigungszapfens 24 parallele exzentrische Sacklochbohrung 36 mündet.

Die Sacklochbohrung 36 dient zur Aufnahme eines Zylinderbolzens 38, der über eine innere Stirnfläche 40 einer den Befestigungszapfen 24 aufnehmenden, an dessen Form angepaßten kegelstumpfförmigen Ausnehmung 42 des Zwischenstücks 16 übersteht und einstückig mit dem Boden der Ausnehmung 42 verbunden ist. Die Sacklochbohrung 36 und der spielfrei in dieselbe passende Zylinderbolzen 38 bilden zusammen eine Verdrehsicherung, die ein Verdrehen des Zwischenstücks 16 auf dem Schaftteil 4 verhindert.

Das Zwischenstück 16 weist konvex gerundete Außenflächen und eine zur Rotationsachse 18 senkrechte ebene obere Stirnfläche 44 auf, auf der sich eine komplementäre untere Stirnfläche des Halteteils 8 abstützt. Über die obere Stirnfläche 44 des Zwischenstücks 16 steht ein zylindrischer Drehzapfen 46 über, der um die Rotationsachse 18 drehbar und in axialer Richtung unverschiebbar in eine entsprechend angepaßte Aufnahmebohrung 48 des Halteteils 8 eingreift. Der Drehzapfen 46 und die Aufnahmebohrung 48 in einem zylindrischen Schaft 50 des Halteteils 8 weisen in ihren einander benachbarten Umfangsflächen an entsprechenden Stellen gegenüberliegende Umfangsnuten 52, 54 auf, in die ein Sicherungsring 56 eingesetzt ist, der ein Herausziehen des Drehzapfens 46 aus der Aufnahmebohrung 48 verhindert, jedoch eine ungehinderte gegenseitige Drehung des Halteteils 8 und des Zwischenstücks 16 um die Rotationsachse 18 ermöglicht, welche eine erste Dreh- oder Schwenkachse 18 der kardanischen Aufhängung bildet.

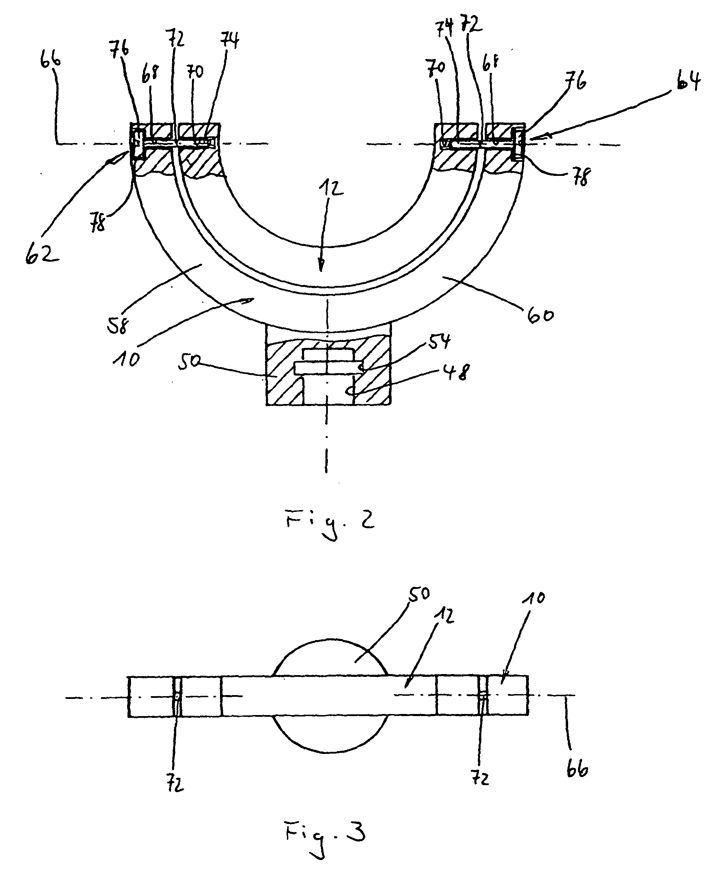

Der Halteteil 8 ist im wesentlichen gabelförmig ausgebildet, wobei die Gabel 10 aus zwei einstückig mit dem Schaft 50 des Halteteils 8 und miteinander verbundenen Gabelteilen 58, 60 besteht, die im wesentlichen in Form eines Viertelkreissektors gebogen sind. Der Querschnitt der Gabelteile 58, 60 kann quadratisch sein, wie in Fig. 3 dargestellt, oder alternativ gerundete Kanten oder Begrenzungsflächen aufweisen.

Am freien Ende der beiden Gabelteile 58, 60 befindet sich jeweils ein Drehlager 62, 64 für das Ringsegment 12, das bei einer vertikalen Ausrichtung der Rotationsachse 18 zur Gabel 10 konzentrisch ist. Die Drehachsen 66 der beiden Drehlager 62, 64 fluchten miteinander und bilden die zweite Dreh- oder Schwenkachse 66 der kardanischen Aufhängung.

Wie in Fig. 2 dargestellt, bestehen die beiden Drehlager 62, 64 jeweils aus einer Querbohrung 68 in den freien Enden der Gabelteile 58, 60, die mit einer gegenüberliegenden Gewindebohrung 70 im Ringsegment 12 fluchtet, wobei jeweils ein Schwenkbolzen 72 mit einem vorderen Gewindeteil 74 in die Gewindebohrung 70 eingeschraubt ist und mit einem hinteren zylindrischen Schaftteil schwenkbar in der Querbohrung 68 eines der Gabelteile 58, 60 gelagert ist. Um einen Austritt der Schwenkbolzen 72 aus ihrem Sitz zu verhindern, kann vor dem Einschrauben des Gewindeteils eine geringe Menge eines biokompatiblen Klebstoffs in die Gewindebohrung 70 gegeben werden. Die Schwenkbolzen 72 weisen einen erweiterten Kopf 76 auf, der zum einen das Einschrauben erleichtert und zum anderen durch sein Anschlagen gegen eine Ringschulter 78 der Querbohrung das Ringsegment 12 und die Gabel 10 in einem im wesentlichen gleichbleibenden Abstand hält.

Das Ringsegment 12 erstreckt sich ebenso wie die Gabel 10 über einen Umfangswinkel von 180 Grad, wobei wie bei dieser die freien Enden zur Anbringung der Drehlager 62, 64 parallel zueinander etwas verlängert sind. Die Dreh- oder Schwenkachse 66 verläuft diametral entlang einer Basis eines von der Gabel 10 bzw. dem Ringsegment 12 umschlossenen Halbkreises.

Der einstückig ausgebildete Sattelteil 6 weist zwei einander gegenüberliegende Sattelhörner 80, 82 auf, zwischen denen sich eine rinnenförmige Sattelmulde 84 erstreckt, die eine Führung für einen Randteil des Beckenknochens (nicht dargestellt) bildet. Die Sattelmulde 84 weist einen im wesentlichen U-förmigen Querschnitt auf, wobei die einander zugewandten parallelen Innenseiten 86, 88 der Sattelhörner 80, 82 Führungsflächen bilden, die gegen entgegengesetzte Knochenoberflächen des in die Sattelmulde 84 eingreifenden Randteils des Beckenknochens bilden. Die Führungsflächen sind untereinander durch eine vom halbzylindrischen Boden 90 der Sattelmulde 84 gebildete Stützfläche verbunden, auf der sich der Beckenknochen im Sattelteil 6 abstützt. Je nachdem, ob der Beckenknochen beweglich im Sattelteil 6 geführt oder gegenüber diesem fixiert werden soll, sind die Führungsflächen 86, 88 und/oder die Stützfläche 90 als polierte, das Anwachsen von Knochen verhindernde oder mit Unebenheiten versehene, das Einwachsen von Knochen fördernde Oberflächen ausgebildet.

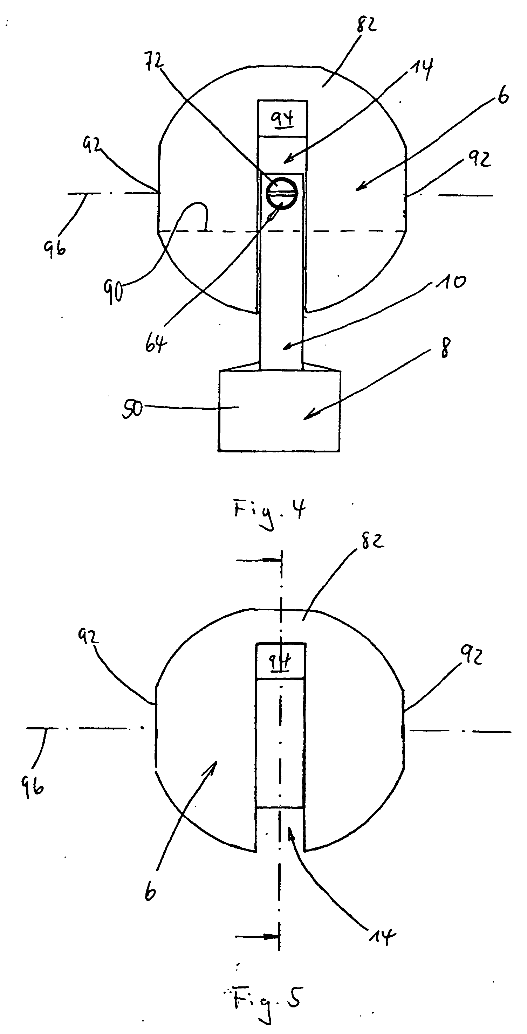

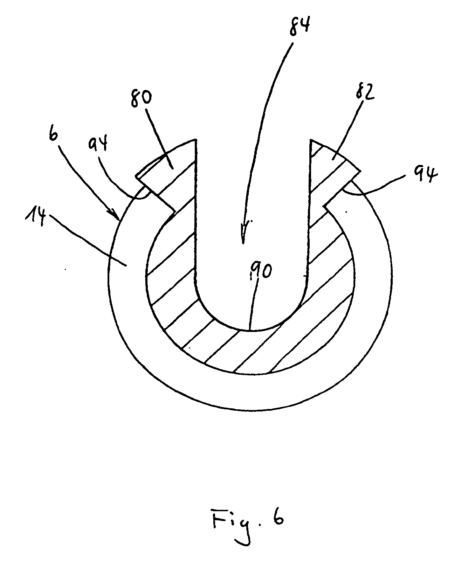

Der Sattelteil 6 weist eine im wesentlichen kugelige äußere Form mit etwas abgeflachten Stirnflächen 92 (Fig. 4 und 5) an den entgegengesetzten Stirnenden der Sattelmulde 84 auf. Auf seiner Unterseite und auf den entgegengesetzten Außenseiten der Sattelhörner 80, 82 ist die ringförmige Führungsnut 14 für das Ringsegment 12 ausgespart, die sich quer zur Längsrichtung der rinnenförmigen Sattelmulde 84 über einen Umfangswinkel von etwa 270 Grad erstreckt und an ihren Stirnenden von ebenen Anschlagflächen 94 begrenzt wird, die in Bezug zur dritten, zwischen den Sattelhörnern 80, 82 hindurch verlaufenden Dreh- und Schwenkachse 96 der kardanischen Aufhängung im wesentlichen radial ausgerichtet sind. Die Führungsnut 14 und das Ringsegment 12 weisen aneinander angepaßte Querschnitte und glatte Oberflächen auf, so daß das Ringsegment 12 reibungsarm in der Führungsnut 14 gleitet. Das Ringsegment 12 oder der Sattelteil 6 kann mit einer z.B. aus Polyethylen bestehenden Beschichtung (nicht dargestellt) versehen sein, um die Reibung zwischen den aufeinander gleitenden Oberflächen zu vermindern. Bei einer etwas dickeren Ausbildung der Beschichtung z.B. in Form einer Einlage im Sattelteil 6 kann die Beschichtung gleichzeitig dazu dienen, den Sattelteil 6 im Ringsegment 12 zu verrasten. Die erfolgt zweckmäßig dadurch, daß man den inneren Umfang des Ringsegments im Unterschied zu dem in der Zeichnung dargestellten Ausführungsbeispiel über einen Winkel von etwas mehr als 180 Grad verlaufen läßt, so daß die Einlage zum gegenseitigen Zusammendrücken des Sattelteils 6 und des Ringsegments 12 elastisch verformt werden muß und somit später als Luxationssicherung dienen kann, die bei einer Zugbeanspruchung ein Lösen des Ringsegments 12 vom Sattelteil 6 verhindert.

Zwei der drei Dreh- oder Schwenkachsen 18, 66, 96 der kardanischen Aufhängung des Sattelteils 6, nämlich die zwischen den Sattelhörnern 80, 82 hindurch in Längsrichtung der Sattelmulde 84 verlaufende Dreh- oder Schwenkachse 96 des Sattelteils 6 und des Ringsegments 12, sowie die quer zur Längsrichtung der Sattelmulde 84 durch die Sattelhörner 80, 82 hindurch verlaufende Dreh- oder Schwenkachse 66 oberhalb der halbzylindrischen Stützfläche 90, so daß der Sattelteil 6 unabhängig von der Richtung einer vom Beckenknochen über die Sattelprothese 2 auf das Bein übertragenen Gewichtskraft gegenüber dem Schaftteil 4 immer in seine stabilste Stellung verschwenkt wird, so daß ein Verkippen unmöglich ist.

Die beiden Dreh- oder Schwenkachsen 66, 96 liegen in zwei Symmetrieebenen des Sattelteils 6, die dieses in Längsrichtung in der Mitte der Sattelmulde 84 und quer dazu in zwei spiegelsymmetrische Hälften unterteilen. Die drei Dreh- oder Schwenkachsen 18, 66, 96 der kardanischen Aufhängung schneiden sich außerdem im Schwerpunkt S des Sattelteils 6, so daß unabhängig von dessen Ausrichtung keine Eigendrehmomente darauf einwirken.

Der Sattelteil 6, das Ringsegment 12, der Halteteil 8, das Zwischenstück 16 und der Schaftteil 4 bestehen aus einem körperverträglichen Implantatmaterial, beispielsweise einer CoCrMo-Legierung und sind lösbar miteinander verbunden, so daß sie als Teilesatz mit jeweils mehreren unterschiedlichen Längenabmessungen aufbewahrt und entsprechend der Anatomie des Patienten zusammengesetzt werden können.

Bei einer Hüftgelenktotaloperation unter Verwendung der Sattelprothese 2 wird nach einer Entfernung der zerstörten oder erkrankten Knochenbereiche und einer Resektion des oberen Endes des Femur zur Schaffung einer ebenen Auflagefläche für die Unterseite 26 des Kragens 22 des Schaftteils 4 zunächst der Markhöhlenkanal des Femur für eine Aufnahme des Schaftes 20 des Schaftteils 4 präpariert. Anschließend wird der Beckenknochen vorbereitet, wobei entweder ein bei der vorangehenden Resektion der zerstörten oder erkrankten Knochenbereiche zurückbleibender passender Randteil oder ein Teil eines Randes einer im Beckenknochen herausgearbeiteten Öffnung zur Aufnahme eines der Sattelhörner 80, 82 so angepaßt werden, daß sie gut mit der von der Sattelmulde 84 gebildeten Führung zusammenpassen. Danach wird ein Zwischenstück 16 mit einer solchen Länge ausgewählt, daß es bei einem probeweisen Einsetzen der Prothese 2 optimal zwischen den Schaftteil 4 und den zuvor mit dem Sattelteil 6 und dem Ringsegment 12 zu einer Einheit zusammengesetzten Halteteil 8 paßt. Dann wird das ausgewählte Zwischenstück 16 mit Hilfe des Sicherungsrings 56 axial unverschiebbar und drehbar mit dem Halteteil 8 verbunden und zuletzt nach dem Verankern des Schaftes 20 im Femur und dem Aufschieben des Sattelteils 6 auf den präparierten Randteil des Beckenknochens das Bein gestreckt und der kegelstumpfförmige Befestigungszapfen 24 so in die Ausnehmung 42 eingeführt, daß der Zylinderbolzen 38 in die Sacklochbohrung 36 gleitet.

## Patentansprüche

1. Sattelprothese für einen Hüftgelenktotalersatz, mit einem in ein Femur einsetzbaren Schaftteil (4) und einem mit dem Schaftteil verbundenen Sattelteil (6), der um mindestens zwei Rotationsachsen gegenüber dem Schaftteil rotationsbeweglich ist und zwischen zwei Sattelhörnern (80, 82) eine Sattelmulde (84) aufweist, die eine Stützfläche (90) für einen Teil eines Beckenknochens bildet, **dadurch gekennzeichnet, daß** mindestens eine der beiden Rotationsachsen (66, 96) oberhalb der Stützflächen (90) angeordnet ist.

2. Sattelprothese nach Anspruch 1, **dadurch gekennzeichnet, daß** die Dreh- oder Schwenkachse (66) quer zur Längsrichtung der Sattelmulde (84) zwei einander gegenüberliegende Sattelhörner (80, 82) des Sattelteils (6) durchsetzt.

3. Sattelprothese nach Anspruch 1 oder 2, **dadurch gekennzeichnet, daß** sich die Dreh- oder Schwenkachse (96) in Längsrichtung der Sattelmulde (84) zwischen zwei einander gegenüberliegenden Sattelhörnern (80, 82) hindurch erstreckt.

4. Sattelprothese nach einem der Ansprüche 1 bis 3, **dadurch gekennzeichnet, daß** die Dreh- oder Schwenkachse (66, 96) in mindestens einer Symmetrieebene des Sattelteils (6) liegt.

5. Sattelprothese nach einem der Ansprüche 1 bis 4, **dadurch gekennzeichnet, daß** die Dreh- oder Schwenkachse (66, 96) durch den Schwerpunkt (100) des Sattelteils (6) verläuft.

6. Sattelprothese nach einem der Ansprüche 1 bis 5, **dadurch gekennzeichnet, daß** der Sattelteil (6) gegenüber dem Schaftteil (4) um zwei unterschiedliche, über der Stützfläche (90) liegende Schwenk- oder Drehachsen (66, 96) rotationsbeweglich ist.

7. Sattelprothese nach Anspruch 6, **dadurch gekennzeichnet, daß** Rotationsbewegungen um die beiden Schwenk- oder Drehachsen (66, 96) unabhängig erfolgen.

8. Sattelprothese nach Anspruch 6 oder 7, **dadurch gekennzeichnet, daß** sich die beiden Schwenk- oder Drehachsen (66, 96) kreuzen.

9. Sattelprothese nach einem der Ansprüche 6 bis 8, **dadurch gekennzeichnet, daß** sich die beiden Schwenk- oder Drehachsen (66, 96) oder Projektionen der beiden Schwenk- oder Drehachsen (66, 96) in eine Ebene unter einem rechten Winkel schneiden.

10. Sattelprothese nach einem der Ansprüche 1 bis 8, **dadurch gekennzeichnet, daß** der Sattelteil (6) gegenüber dem Schaftteil (4) kardanisch um drei Achsen (18, 66, 96) verschwenkbar ist.

11. Sattelprothese nach Anspruch 10, **dadurch gekennzeichnet, daß** die drei Achsen (18, 66, 96) von den zwei über der Stützfläche (90) liegenden Schwenk- oder Drehachsen (66, 96) und einer im wesentlichen in Beinlängsrichtung verlaufenden Rota-tionsachse (18) für eine Außen/Innenrotation des Beins gebildet werden.

12. Sattelprothese nach Anspruch 10 oder 11, **dadurch gekennzeichnet, daß** sich die drei Achsen (18, 66, 96) jeweils unter rechten Winkeln schneiden.

13. Sattelprothese nach einem der Ansprüche 10 bis 12, **dadurch gekennzeichnet, daß** der Sattelteil (6) an einem mit dem Schaftteil (4) verbundenen oder verbindbaren Halteteil (8) um die Dreh- oder Schwenkachsen (66, 96) rotationsbeweglich aufgehängt ist.

14. Sattelprothese nach einem der Ansprüche 11 bis 13, **dadurch gekennzeichnet, daß** der Halteteil (8) gegenüber dem Schaftteil (4) um die Rotationsachse (18) drehbar ist.

15. Sattelprothese nach einem der Ansprüche 11 bis 14, **dadurch gekennzeichnet, daß** der Halteteil (8) einen um die Rotationsachse (18) drehbaren Schaft und eine über den Schaft überstehende Gabel (10) aufweist.

16. Sattelprothese nach einem der Ansprüche 13 bis 15, **gekennzeichnet durch** ein verschwenkbar am Halteteil (8) gelagertes und in einer ringförmigen Führungsnut (14) des Sattelteil (6) verschiebbares Ringsegment (12).

17. Sattelprothese nach Anspruch 16, **dadurch gekennzeichnet, daß** sich das Ringsegment (12) in der ringförmigen Führungsnut (14) unter Verschwenken um die Dreh- oder Schwenkachse (96) verschiebt, welche sich zwischen den beiden Sattelhörnern (80, 82) hindurch erstreckt.

18. Sattelprothese nach Anspruch 16 oder 17, **dadurch gekennzeichnet, daß** sich die Führungsnut (14) über einen Winkel von 260 bis 310 Grad und vorzugsweise von 270 bis 300 Grad über die Unterseite des Sattelteils (6) erstreckt.

19. Sattelprothese nach einem der Ansprüche 16 bis 18, **dadurch gekennzeichnet, daß** die Führungsnut (14) in einer kugelig gerundeten Außenfläche des Sattelteils (6) ausgespart ist.

20. Sattelprothese nach einem der Ansprüche 16 bis 19, **dadurch gekennzeichnet, daß** das Ringsegment (12) verschiebbar in der Führungsnut (14) befestigbar ist.

21. Sattelprothese nach einem der Ansprüche 16 bis 20, **dadurch gekennzeichnet, daß** sich das Ringsegment (12) über einen Umfangswinkel von 150 bis 190 Grad erstreckt.

22. Sattelprothese nach Anspruch 20, **dadurch gekennzeichnet, daß** sich das Ringsegment (12) über einen Umfangswinkel von 150 bis 180 Grad erstreckt, und daß der Sattelteil (6) mit der Führungsnut (14) lose auf das Ringsegment (12) aufgelegt ist.

23. Sattelprothese nach Anspruch 20, **dadurch gekennzeichnet, daß** sich das Ringsegment (12) über einen Umfangswinkel von etwas mehr als 180 Grad erstreckt und in der Führungsnut (14) des Sattelteils (6) einrastbar ist.

24. Sattelprothese nach einem der Ansprüche 1 bis 23, **dadurch gekennzeichnet, daß** die Sattelmulde (84) des Sattelteils (6) eine Führung für den Beckenknochen bildet.

25. Sattelprothese nach Anspruch 24, **dadurch gekennzeichnet, daß** gegenüberliegende Innenflächen (86, 88) der Sattelhörner (80, 82) Führungsflächen für entgegengesetzte Knochenoberflächen des Beckenknochens bilden.

26. Sattelprothese nach einem der Ansprüche 1 bis 25, **dadurch gekennzeichnet, daß** die Stützfläche von einem konkav gewölbten Boden (90) der Sattelmulde (84) gebildet wird.

27. Sattelprothese nach einem der Ansprüche 1 bis 26, **dadurch gekennzeichnet, daß** die Sattelmulde (84) rinnenförmig ausgebildet ist und einen im wesentlichen U-förmigen Querschnitt aufweist.

28. Sattelprothese nach einem der Ansprüche 1 bis 27, **dadurch gekennzeichnet, daß** die Stützfläche teilzylindrisch nach außen gewölbt ist.

29. Sattelprothese nach einem der Ansprüche 23 bis 28, **dadurch gekennzeichnet, daß** mindestens ein Teil der Führungsflächen und/oder der Stützfläche poliert ist.

30. Sattelprothese nach einem der Ansprüche 23 bis 28, **dadurch gekennzeichnet, daß** mindestens ein Teil der Führungsflächen und/oder der Stützfläche als Knocheneinwachsoberfläche ausgebildet ist.

31. Sattelprothese nach einem der vorangehenden Ansprüche, **dadurch gekennzeichnet, daß** der Sattelteil (6), das Ringsegment (12), der Halteteil (8) und der Schaftteil (4) mindestens zum Teil lösbar miteinander verbunden sind.

32. Sattelprothese nach Anspruch 31, **gekennzeichnet durch** eine zwischen dem Ringsegment (12) und dem Sattelteil (6) und/oder zwischen dem Halteteil (8) und dem Schaftteil (4) angeordnete Luxationssicherung.

33. Sattelprothese nach Anspruch 30 oder 32, **gekennzeichnet durch** ein zwischen dem Schaftteil (4) und dem Halteteil (8) angeordnetes Zwischenstück (16).

34. Sattelprothese nach Anspruch 33, **dadurch gekennzeichnet, daß** der Halteteil (8) drehbar und axial unverschiebbar mit dem Zwischenstück (16) verbunden ist.

35. Sattelprothese nach Anspruch 33 oder 34, **dadurch gekennzeichnet, daß** das Zwischenstück (16) einen nach oben überstehenden Drehzapfen (46) aufweist, der in eine Aufnahmebohrung (48) im Halteteil (8) eingreift.

36. Sattelprothese nach Anspruch 35, **gekennzeichnet durch** einen in gegenüberliegende Umfangsnuten (52, 54) der Aufnahmebohrung (48) und des Drehzapfens (46) eingesetzten Sicherungsring (56).

37. Sattelprothese nach einem der Ansprüche 30 bis 36, **dadurch gekennzeichnet, daß** das Zwischenstück (16) eine kegelstumpfförmige Ausnehmung (42) für einen kegelstumpfförmigen Befestigungszapfen (24) des Schaftteils (4) aufweist.

38. Sattelprothese nach Anspruch 37, **gekennzeichnet durch** eine Verdrehsicherung, die ein Verdrehen des Befestigungszapfens (24) in der Ausnehmung (42) verhindert.

39. Sattelprothese nach Anspruch 38, **dadurch gekennzeichnet, daß** die Verdrehsicherung eine in einem Abstand von einer Mittelachse (32) des Befestigungszapfens (24) angeordnete und zur Mittelachse (32) parallele Sacklochbohrung (36) sowie einen im gleichen Abstand von einer Mittelachse der Ausnehmung (42) angeordneten und zur Mittelachse parallelen Zylinderbolzen (38) umfaßt, der spielfrei in die Sacklochbohrung (36) paßt.

40. Sattelteil (6) für eine Sattelprothese nach einem der Ansprüche 1 bis 39, der eine Stützfläche (90) aufweist und der einen Teil eines Gelenks (12, 14) mit einer Rotationsachse (96) bildet, **dadurch gekennzeichnet, daß** die Rotationsachse (96) oberhalb der Stützfläche (90) des Sattelteils (6) liegt.

## Claims

1. A saddle prosthesis for total replacement of a hip joint, with a shaft part (4) which can be inserted into the femur, and with a saddle part (6) which is connected to the shaft part and which can undergo rotational movement about at least two axes of rotation relative to the shaft part, and between two saddle horns (80,82) has a saddle depression (84) which forms a support surface (90) for a part of a pelvic bone, **characterised in that** at least one of the two axes of rotation (66,96) is disposed above the support surfaces (90).

2. A saddle prosthesis according to Claim 1, **characterised in that** transversely to the longitudinal direction of the saddle depression (84) the axis of rotation or pivot axis (66) passes through two mutually opposite saddle horns (80,82) of the saddle part (6).

3. A saddle prosthesis according to Claim 1 or 2, **characterised in that** the axis of rotation or pivot axis (96) extends in the longitudinal direction of the saddle depression (84) between two mutually opposite saddle horns (80,82).

4. A saddle prosthesis according to any one of Claims 1 to 3, **characterised in that** the axis of rotation or pivot axis (66,96) lies on at least one plane of symmetry of the saddle part (6).

5. A saddle prosthesis according to any one of Claims 1 to 4, **characterised in that** the axis of rotation or pivot axis (66,96) extends through the centre of gravity (100) of the saddle part (6).

6. A saddle prosthesis according to any one of Claims 1 to 5, **characterised in that** the saddle part (6) can undergo rotational movement relative to the shank part (4) about two different axes of rotation or pivot axes (66,96) situated above the support surface (90).

7. A saddle prosthesis according to Claim 6, **characterised in that** the rotational movements take place independently about the two axes of rotation or pivot axes (66,96).

8. A saddle prosthesis according to Claim 6 or 7, **characterised in that** the two axes of rotation or pivot axes (66,96) intersect one another.

9. A saddle prosthesis according to any one of Claims 6 to 8, **characterised in that** the two axes of rotation or pivot axes (66,96) or projections of the two axes of rotation or pivot axes (66,96) intersect in one plane at a right angle.

10. A saddle prosthesis according to any one of Claims 1 to 8, **characterised in that** the saddle part (6) is pivotable about three axes (18,66,96) relative to the shank part (4) in the manner of a cardan joint.

11. A saddle prosthesis according to Claim 10, **characterised in that** the three axes (18,66,96) are formed by the two axes of rotation or pivot axes (66,96) situated above the support surface (90) and one axis of rotation (18) extending substantially in the longitudinal direction of the bone for outward/inward rotation of the bone.

12. A saddle prosthesis according to Claim 10 or 11, **characterised in that** the three axes (18,66,96) intersect one another at right angles.

13. A saddle prosthesis according to any one of Claims 10 to 12, **characterised in that** the saddle part (6) is suspended from a retaining member (8) which is connected or can be connected to the shank part (4) so as to be able to undergo rotational movement about the axes of rotation or pivot axes (66,96).

14. A saddle prosthesis according to any one of Claims 11 to 13, **characterised in that** the retaining member (8) is rotatable relative to the shank part (4) about the axis of rotation (18).

15. A saddle prosthesis according to any one of Claims 11 to 14, **characterised in that** the retaining member (8) has a shank rotatable about the axis of rotation (18) and a yoke (10) projecting beyond the shank.

16. A saddle prosthesis according to any one of Claims 13 to 15, **characterised by** an annular segment (12) mounted pivotably on the retaining member (8) and displaceably in an annular guide slot (14) of the saddle part (6).

17. A saddle prosthesis according to Claim 16, **characterised in that** the annular segment (12) is displaced in the annular guide slot (14) and is pivoted about the axis of rotation or pivot axis (96) which extends through between the two saddle horns (80,82).

18. A saddle prosthesis according to Claim 16 or 17, **characterised in that** the guide slot (14) extends through an angle of from 260 to 310 degrees and, preferably, from 270 to 300 degrees above the underside of the saddle part (6).

19. A saddle prosthesis according to any one of Claims 16 to 18, **characterised in that** the guide slot (14) is recessed in a spherically rounded outer surface of the saddle part (6).

20. A saddle prosthesis according to any one of Claims 16 to 19, **characterised in that** the annular segment (12) can be fastened displaceably in the guide slot (14).

21. A saddle prosthesis according to any one of Claims 16 to 20, **characterised in that** the annular segment (12) extends through an angle at circumference of from 150 to 190 degrees.

22. A saddle prosthesis according to Claim 20, **characterised in that** the annular segment (12) extends through an angle at circumference of from 150 to 180 degrees, and **in that** the saddle part (6) with the guide slot (14) is placed loosely on the annular segment (12).

23. A saddle prosthesis according to 20, **characterised in that** the annular segment (12) extends through an angle at circumference of slightly more than 180 degrees and can engage in the guide slot (14) of the saddle part (6).

24. A saddle prosthesis according to any one of Claims 1 to 23, **characterised in that** the saddle depression (84) of the saddle part (6) forms a guide for the pelvic bone.

25. A saddle prosthesis according to Claim 24, **characterised in that** the opposing inner surfaces (86,88) of the saddle horns (80,82) form guide surfaces for opposite bone surfaces of the pelvic bone.

26. A saddle prosthesis according to any one of Claims 1 to 25, **characterised in that** the support surface is formed by a concavely curved base (90) of the saddle depression (84).

27. A saddle prosthesis according to any one of Claims 1 to 26, **characterised in that** the saddle depression (84) is channel-shaped and is of substantially U-shaped cross-section.

28. A saddle prosthesis according to any one of Claims 1 to 27, **characterised in that** the support surface is curved partly cylindrically outwards.

29. A saddle prosthesis according to any one of Claims 23 to 28, **characterised in that** at least one part of the guide surfaces and/or of the support surface is polished.

30. A saddle prosthesis according to any one of Claims 23 to 28, **characterised in that** at least one part of the guide surfaces and/or of the support surface is in the form of a surface for the growing-in of bone.

31. A saddle prosthesis according to any one of the preceding Claims, **characterised in that** the saddle part (6), the annular segment (12), the retaining member (8) and the shank part (4) are joined together at least partly detachably.

32. A saddle prosthesis according to Claim 31, **characterised by** a dislocation protection means disposed between the annular segment (12) and the saddle part (6) and/or between the retaining part (8) and the shank part (4).

33. A saddle prosthesis according to Claim 30 or 32, **characterised by** a connecting piece (16) between the shank part (4) and the retaining member (8).

34. A saddle prosthesis according to Claim 33, **characterised in that** the retaining member (8) is connected rotatably and axially non-displaceably to the connecting piece (16).

35. A saddle prosthesis according to Claim 33 or 34, **characterised in that** the connecting piece (16) has an upwardly projecting pivot (46) which engages in a seating bore (48) in the retaining member (8).

36. A saddle prosthesis according to Claim 35, **characterised by** a retaining ring (56) inserted into opposing circumferential grooves (52,54) of the seating bore (48) and of the pivot (46) [sic].

37. A saddle prosthesis according to any ones of Claims 30 to 36, **characterised in that** the connecting piece (16) has a frustoconical recess (42) for a frustoconical securing peg (24) of the shank part (4).

38. A saddle prosthesis according to Claim 37, **characterised by** an antirotational feature which prevents rotation of the securing peg (24) in the recess (42).

39. A saddle prosthesis according to Claim 38, **characterised in that** the antirotational feature comprises a blind bore (36) disposed at a distance from a centre axis (32) of the securing peg (24) and parallel to the centre axis (32), and it also comprises a cylindrical pin (38) which is disposed at an equal distance from a centre axis of the recess (42) and parallel to the centre axis, which pin fits without clearance into the blind bore (36).

40. A saddle part (6) for a saddle prosthesis according to any one of Claims 1 to 39, which has a support surface (90) and which forms part of a joint (12,14) with an axis of rotation (96), **characterised in that** the axis of rotation (96) is situated above the support surface (90) of the saddle part (6).

## Revendications

1. Prothèse en forme de selle pour le remplacement total d'une articulation de la hanche, comportant une partie formant tige (4) pouvant être insérée dans un fémur et une partie en selle (6) reliée à la partie formant tige et qui est mobile en rotation par rapport à la partie formant tige, autour d'au moins deux axes de rotation, et présente, entre deux cornes de selle (80, 82), un creux de selle (84) qui forme une surface d'appui (90) pour une partie d'un os du bassin, **caractérisée en ce qu'**au moins l'un des deux axes de rotation (66, 96) est disposé au-dessus des surfaces d'appui (90).

2. Prothèse en forme de selle selon la revendication 1, **caractérisée en ce que** l'axe de rotation ou de pivotement (66) traverse, transversalement à la direction longitudinale du creux de selle (84), deux cornes de selle (80, 82) opposées l'une à l'autre de la partie en selle (6).

3. Prothèse en forme de selle selon la revendication 1 ou 2, **caractérisée en ce que** l'axe de rotation ou de pivotement (96) s'étend dans la direction longitudinale du creux de selle (84) entre deux cornes de selle (80, 82) opposées l'une à l'autre.

4. Prothèse en forme de selle selon l'une des revendications 1 à 3, **caractérisée en ce que** l'axe de rotation ou de pivotement (66, 96) se situe dans au moins un plan de symétrie de la partie en selle (6).

5. Prothèse en forme de selle selon l'une des revendications 1 à 4, **caractérisée en ce que** l'axe de rotation ou de pivotement (66, 96) passe par le centre de gravité (100) de la partie en selle (6).

6. Prothèse en forme de selle selon l'une des revendications 1 à 5, **caractérisée en ce que** la partie en selle (6) est mobile en rotation par rapport à la partie formant tige (4), autour de deux axes de pivotement ou de rotation (66, 96) différents, situés au-dessus de la surface d'appui (90).

7. Prothèse en forme de selle selon la revendication 6, **caractérisée en ce que** les mouvements de rotation autour des deux axes de pivotement ou de rotation (66, 96) s'effectuent indépendamment.

8. Prothèse en forme de selle selon la revendication 6 ou 7, **caractérisée en ce que** les deux axes de pivotement ou de rotation (66, 96) se croisent.

9. Prothèse en forme de selle selon l'une des revendications 6 à 8, **caractérisée en ce que** les deux axes de pivotement ou de rotation (66, 96) ou des projections des deux axes de pivotement ou de rotation (66, 96) se coupent dans un plan, suivant un angle droit.

10. Prothèse en forme de selle selon l'une des revendications 1 à 8, **caractérisée en ce que** la partie en selle (6) peut pivoter par rapport à la partie formant tige (4) à la manière d'un cardan, autour de trois axes (18, 66, 96).

11. Prothèse en forme de selle selon la revendication 10, **caractérisée en ce que** les trois axes (18, 66, 96) sont formés par les deux axes de pivotement ou de rotation (66, 96), situés au-dessus de la surface d'appui (90), et par un axe de rotation (18), s'étendant sensiblement dans la direction longitudinale de la jambe, pour une rotation extérieure/intérieure de la jambe.

12. Prothèse en forme de selle selon la revendication 10 ou 11, **caractérisée en ce que** les trois axes (18, 66, 96) se coupent respectivement suivant des angles droits.

13. Prothèse en forme de selle selon l'une des revendications 10 à 12, **caractérisée en ce que** la partie en selle (6) est suspendue mobile en rotation autour de l'axe de rotation ou de pivotement (66, 96) à une partie de maintien (8) reliée ou pouvant être reliée à la partie formant tige (4).

14. Prothèse en forme de selle selon l'une des revendications 11 à 13, **caractérisée en ce que** la partie de maintien (8) peut tourner par rapport à la partie formant tige (4) autour de l'axe de rotation (18).

15. Prothèse en forme de selle selon l'une des revendications 11 à 14, **caractérisée en ce que** la partie de maintien (8) comporte une tige pouvant tourner autour de l'axe de rotation (18) et une fourche (10) dépassant de la tige.

16. Prothèse en forme de selle selon l'une des revendications 13 à 15, **caractérisée par** un segment d'anneau (12) monté pivotant sur la partie de maintien (8) et pouvant coulisser dans une rainure de guidage (14) de forme annulaire de la partie en selle (6).

17. Prothèse en forme de selle selon la revendication 16, **caractérisée en ce que** le segment d'anneau (12) coulisse dans la rainure de guidage (14) de forme annulaire avec pivotement autour de l'axe de rotation ou de pivotement (96) qui s'étend à travers les deux cornes de selle (80, 82).

18. Prothèse en forme de selle selon la revendication 16 ou 17, **caractérisée en ce que** la rainure de guidage (14) s'étend sur un angle de 260 à 310° et de préférence de 270 à 300° sur la face inférieure de la partie en selle (6).

19. Prothèse en forme de selle selon l'une des revendications 16 à 18, **caractérisée en ce que** la rainure de guidage (14) est découpée dans une surface extérieure arrondie en forme de sphère de la partie en selle (6).

20. Prothèse en forme de selle selon l'une des revendications 16 à 19, **caractérisée en ce que** le segment d'anneau (12) peut être fixé coulissant dans la rainure de guidage (14).

21. Prothèse en forme de selle selon l'une des revendications 16 à 20, **caractérisée en ce que** le segment d'anneau (12) s'étend sur un angle périphérique de 150 à 190°.

22. Prothèse en forme de selle selon la revendication 20, **caractérisée en ce que** le segment d'anneau (12) s'étend sur un angle périphérique de 150 à 180°, et **en ce que** la partie en selle (6) avec la rainure de guidage (14) est placée librement sur le segment d'anneau (12).

23. Prothèse en forme de selle selon la revendication 20, **caractérisée en ce que** le segment d'anneau (12) s'étend sur un angle périphérique légèrement supérieur à 180° et peut s'accrocher dans la rainure de guidage (14) de la partie en selle (6).

24. Prothèse en forme de selle selon l'une des revendications 1 à 23, **caractérisée en ce que** le creux de selle (84) de la partie en selle (6) forme un guide pour l'os du bassin.

25. Prothèse en forme de selle selon la revendication 24, **caractérisée en ce que** des surfaces intérieures (86, 88) opposées des cornes de selle (80, 82) forment des surfaces de guidage pour des surfaces opposées de l'os du bassin.

26. Prothèse en forme de selle selon l'une des revendications 1 à 25, **caractérisée en ce que** la surface d'appui est formée par un fond (90) concave du creux de selle (84).

27. Prothèse en forme de selle selon l'une des revendications 1 à 26, **caractérisée en ce que** le creux de selle (84) est réalisé en forme de rigole et présente une section transversale sensiblement en U.

28. Prothèse en forme de selle selon l'une des revendications 1 à 27, **caractérisée en ce que** la surface d'appui est bombée vers l'extérieur de manière partiellement cylindrique.

29. Prothèse en forme de selle selon l'une des revendications 23 à 28, **caractérisée en ce qu'**au moins une partie des surfaces de guidage et/ou de la surface d'appui est polie.

30. Prothèse en forme de selle selon l'une des revendications 23 à 28, **caractérisée en ce qu'**au moins une partie des surfaces de guidage et/ou de la surface d'appui est réalisée comme surface de croissance de l'os.

31. Prothèse en forme de selle selon l'une des revendications précédentes, **caractérisée en ce que** la partie en selle (6), le segment d'anneau (12), la partie de maintien (8) et la partie formant tige (4) sont reliés entre eux de manière séparable, au moins en partie.

32. Prothèse en forme de selle selon la revendication 31, **caractérisée par** une protection contre les luxations disposée entre le segment d'anneau (12) et la partie en selle (6) et/ou entre la partie de maintien (8) et la partie formant tige (4).

33. Prothèse en forme de selle selon la revendication 30 ou 32, **caractérisée par** une pièce intermédiaire (16) disposée entre la partie formant tige (4) et la partie de maintien (8).

34. Prothèse en forme de selle selon la revendication 33, **caractérisée en ce que** la partie de maintien (8) est reliée tournante et axialement non coulissante à la pièce intermédiaire (16).

35. Prothèse en forme de selle selon la revendication 33 ou 34, **caractérisée en ce que** la pièce intermédiaire (16) comporte un tourillon (46) dépassant vers le haut qui s'engage dans un perçage de réception (48) de la partie de maintien (8).

36. Prothèse en forme de selle selon la revendication 35, **caractérisée par** un anneau de blocage (56) inséré dans des rainures périphériques (52, 54) opposées du perçage de réception (48) et du tourillon (46).

37. Prothèse en forme de selle selon l'une des revendications 30 à 36, **caractérisée en ce que** la pièce intermédiaire (16) présente un évidement (42) en forme de tronc de cône pour un doigt de fixation (24) de forme tronconique de la partie formant tige (4).

38. Prothèse en forme de selle selon la revendication 37, **caractérisée par** un blocage en rotation qui empêche une rotation du doigt de fixation (24) dans l'évidement (42).

39. Prothèse en forme de selle selon la revendication 38, **caractérisée en ce que** le blocage en rotation comprend un trou borgne (36) disposé à distance d'un axe médian (32) du doigt de fixation (24) et parallèle à l'axe médian (32), ainsi qu'une tige cylindrique (38) disposée à même distance d'un axe médian de l'évidement (42) et parallèle à l'axe médian, lequel s'ajuste sans jeu dans le trou borgne (36).

40. Partie en selle (6) pour une prothèse en forme de selle selon l'une des revendications 1 à 39 qui présente une surface d'appui (90) et forme une partie d'une articulation (12, 14) avec un axe de rotation (96), **caractérisée en ce que** l'axe de rotation (96) se situe au-dessus de la surface d'appui (90) de la partie en selle (6).
